# EUROPEAN PATENT APPLICATION

(11) **EP 3 824 852 A1**
(43) Date of publication of application: **26.05.2021**
(21) Application number: 19210851.2
(22) Date of filing: 22.11.2019
(51) Int. Cl.: A61F 5/445

(54) **ARTIFICIAL STOMA DEVICE**

(71) Applicant: Enterme Medicine International Co., Ltd., Kaohsiung City 81366 (TW)
(72) Inventor: Chang, Yi-Hsin, 81366 Kaohsiung City (TW)
(74) Representative: Pawlyn, Anthony Neil

(57) **Abstract**

An artificial stoma device includes a first unit (3) and a second unit (4) adapted to be respectively inserted into an upstream section (911) and a downstream section (912) of an intestine (91) via an opening (920) of a skin tissue (92) and via a radial side opening (910) of the intestine (91) . The first unit (3) includes a hollow member (30) that has an inlet portion (31) and an outlet portion (32). The inlet portion (31) has an inlet opening (310) at a distal end thereof that is adapted for facing the upstream section (911) of the intestine (91). The outlet portion (32) is connected to the inlet portion (31), and has an outlet opening (320) that is opposite to and in spatial communication with the inlet opening (310) and that is adapted to be exposed from the skin tissue (92) via the opening (920).

## Description

The disclosure relates to an artificial organ, and more particularly to an artificial stoma device.

An artificial stoma is an opening of a severed intestine formed as a substitute for excretion when a part of the intestine is cut off due to lesions in the intestine of a patient. In general, an artificial stoma serves as a replacement of a downstream section of the intestine after it is removed, or as a temporary replacement of the downstream section of the intestine as it is recovering.

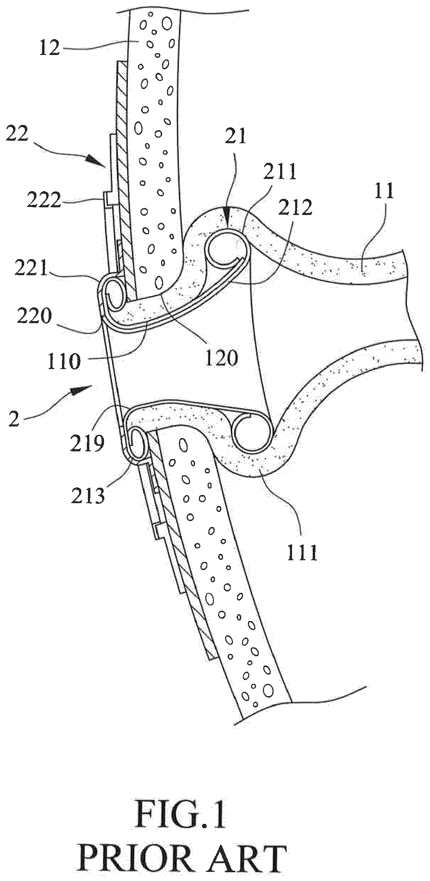

Referring to FIG. 1, a conventional artificial stoma device 2 is adapted to be installed in a first opening 110 formed in a severed intestine 11 and in a second opening 120 formed in a human body 12. The intestine 11 extends from the inside to the outside of the second opening 120 in such a way that the first opening 110 faces outwardly. The artificial stoma device 2 includes a brace member 21 adapted to be disposed in the first opening 110 of the intestine 11, and a positioning member 22 connected to an outer portion of the brace member 21.

The brace member 21 is hollow and includes an inner ring 211 that is adapted to abut against an inner wall of the intestine 11, an extension tube 212 that is connected to the inner ring 211 and that has a cross-section decreasing in a direction away from the inner ring 211, and an outer ring 213 that is connected to an end of the extension tube 212 opposite to the inner ring 211, and that defines an aperture 219. The positioning member 22 is adapted to be attached to the skin of the human body 12, and includes an annular portion 221 that defines an annular hole 220, and a plate portion 222 that is adapted to be attached to the human body 12. The inner ring 211 has a diameter larger than that of the outer ring 213. When the brace member 21 is disposed inside the intestine 11, a portion 111 of the intestine 11 is expanded by the inner ring 211. The outer ring 213 surrounds the first opening 110 of the intestine 11, and the aperture 219, the intestinal tract of the intestine 11, and the external environment are in spatial communication with each other. The outer ring 213 abuts against the outside of the human body 12, while the inner ring 211 abuts the portion 111 of the intestine 11 against the inner surface of the human body 12, so that the intestine 11 is positioned at the second opening 120. Then, the annular portion 221 of the position member 22 is attached to the outer ring 213 of the brace member 21, while the plate portion 222 is attached to the peripheral area of the second opening 120, so that the brace member 21 is positioned by the positioning member 22. At this point, the aperture 219 is spatially communicated with the space outside the human body 12 via the annular hole 220, and excrement transported by the intestine 11 can be discharged via the aperture 219.

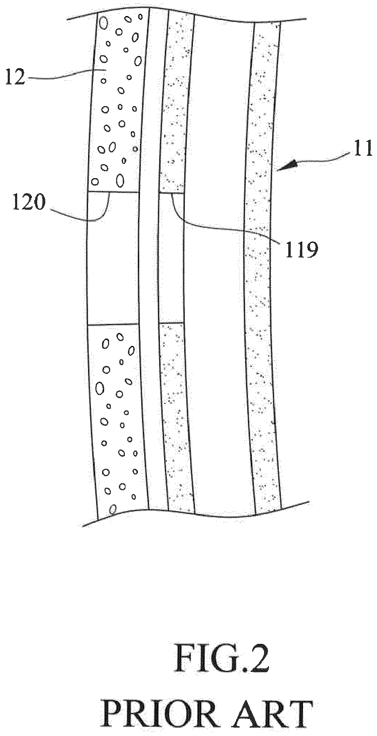

However, besides the abovementioned approach for treating the severed intestine 11, in cases of temporary disposal, a setup in FIG. 2 may be adopted in order to prevent the downstream section of the intestine 11 from becoming necrotic, that is, forming a side opening 119 in the wall of the intestine 11 instead of severing the intestine 11 so that the intestine 11 may continue its normal physiological circulation. When the downstream section of the intestine 11 relative to the side opening 119 has fully recovered, the intestine 11 may be returned to its normal functioning state. However, the artificial stoma device 2 shown in FIG. 1 is not applicable to the setup shown in FIG. 2. If the intestine 11 is directly sutured to the human body 12 such that the side opening is directly connected to the second opening 120 to form an artificial stoma, the excrement may still be transported from the upstream section to the downstream section of the intestine 11 and affect the recovery of the intestine 11. Thus, it is imperative to provide an artificial stoma device that is applicable to the setup shown in FIG. 2.

Therefore, the object of the disclosure is to provide an artificial stoma device that can alleviate at least one of the drawbacks of the prior art.

According to the disclosure, the artificial stoma device is adapted to be installed in an abdomen. The abdomen contains an intestine which is formed with a radial side opening, and has a skin tissue which is formed with an opening being registered with the radial side opening. The intestine has an upstream section that is disposed upstream of the radial side opening and a downstream section that is disposed downstream of the radial side opening. The artificial stoma device includes a first unit and a second unit.

The first unit is adapted to be inserted into the upstream section of the intestine via the opening of the skin tissue and the radial side opening of the intestine. The first unit includes a hollow member that has an inlet portion and an outlet portion.

The inlet portion is adapted to be disposed in the upstream section of the intestine, and has an inlet opening at a distal end thereof that is adapted for facing the upstream section of the intestine.

The outlet portion is adapted to be disposed at the opening of the skin tissue and the radial side opening of the intestine, is connected to the inlet portion, and has an outlet opening that is opposite to and in spatial communication with the inlet opening and that is adapted to be exposed from the skin tissue via the opening.

The second unit is connected to the first unit, and includes an insertion member that is adapted to be inserted into the downstream section of the intestine via the opening of the skin tissue and the radial side opening of the intestine.

Other features and advantages of the disclosure will become apparent in the following detailed description of the embodiments with reference to the accompanying drawings, of which:
FIG. 1 is a fragmentary sectional view illustrating a conventional artificial stoma device connecting an intestine to a human abdomen;
FIG. 2 is a fragmentary sectional view illustrating a setup of forming a side opening in the intestine;
FIG. 3 is an exploded sectional view of a first embodiment of the artificial stoma device according to the disclosure;
FIG. 4 is a fragmentary sectional view illustrating the installation process of the first embodiment in an abdomen;
FIG. 5 is an exploded sectional view of a second embodiment of the artificial stoma device according to the disclosure;
FIG. 6 is an exploded sectional view of a variation of the second embodiment;
FIG. 7 is a fragmentary partly sectional view illustrating a third embodiment of the artificial stoma device according to the disclosure and the installation process thereof;
FIG. 8 is a fragmentary partly sectional view illustrating a fourth embodiment of the artificial stoma device according to the disclosure and the installation process thereof;
FIG. 9 is a partly exploded sectional view of a variation of the fourth embodiment;
FIG. 10 is a partly exploded sectional view of another variation of the fourth embodiment; and
FIG. 11 is a fragmentary partly sectional view illustrating a fifth embodiment of the artificial stoma device according to the disclosure and the installation process thereof.

Before the present invention is described in greater detail, it should be noted that where considered appropriate, reference numerals or terminal portions of reference numerals have been repeated among the figures to indicate corresponding or analogous elements, which may optionally have similar characteristics.

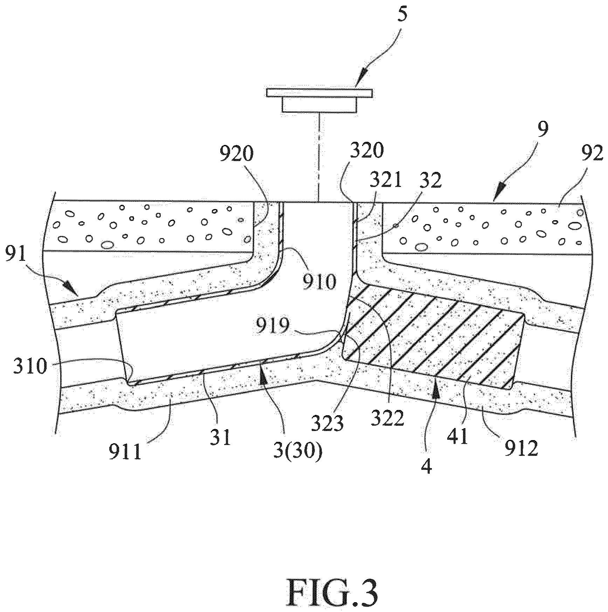

Referring to FIG. 3, a first embodiment of an artificial stoma device according to the present disclosure is adapted to be installed in an abdomen 9 of a patient. The abdomen 9 contains an intestine 91 which is formed with a radial side opening 910, and has a skin tissue 92 which is formed with an opening 920 being registered with the radial side opening 910. The intestine 91 has an upstream section 911 that is disposed upstream of the radial side opening 910 and a downstream section 912 that is disposed downstream of the radial side opening 910. The artificial stoma device of the first embodiment includes a first unit 3, a second unit 4 and a cover unit 5.

The first unit 3 is adapted to be inserted into the upstream section 911 of the intestine 91 via the opening 920 of the skin tissue 92 and the radial side opening 910 of the intestine 91. The first unit 3 includes a hollow member 30 that has an inlet portion 31 and an outlet portion 32.

The inlet portion 31 is adapted to be disposed in the upstream section 911 of the intestine 91, and has an inlet opening 310 at a distal end thereof that is adapted for facing the upstream section 911 of the intestine 91.

The outlet portion 32 is adapted to be disposed at the opening 920 of the skin tissue 92 and the radial side opening 910 of the intestine 91, and is connected to the inlet portion 31. The outlet portion 32 has an outlet opening 320 and a connecting segment 321. The outlet opening 320 is opposite to and in spatial communication with the inlet opening 310 of the inlet portion 31, and is adapted to be exposed from the skin tissue 92 via the opening 920. The connecting segment 321 is adapted to be adjacent to the downstream section 912 of the intestine 91, and has a linking section 322 and a clamping section 323. The linking section 322 is connected to the second unit 4, and the clamping section 323 cooperates with the second unit 4 for clamping a portion 919 of the intestine 91 therebetween.

The second unit 4 includes an insertion member 41 that is solid, and that is adapted to be inserted into the downstream section 912 of the intestine 91 via the opening 920 of the skin tissue 92 and the radial side opening 910 of the intestine 91. In the present embodiment, the insertion member 41 of the second unit 4 and the connecting segment 321 of the first unit 3 are molded as one piece.

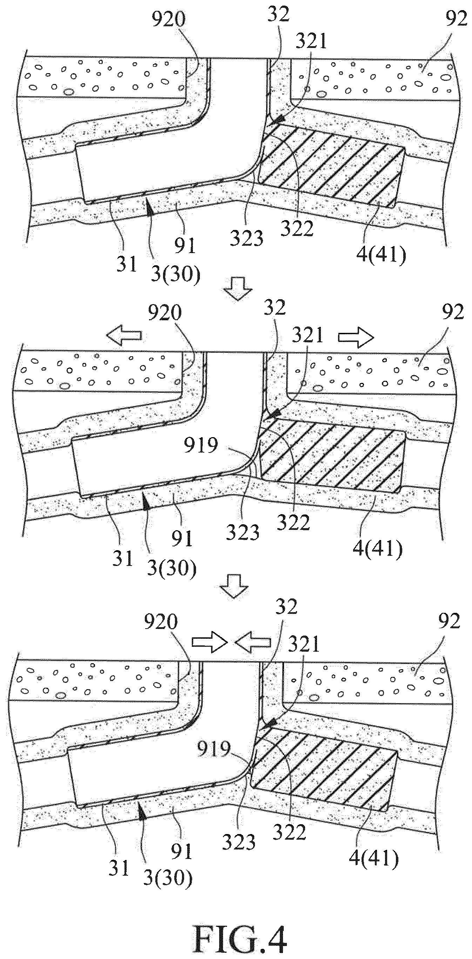

Referring to FIG. 4 in conjunction with FIG. 3, during installation of the artificial stoma device of the present embodiment, after the first and second units 3, 4 are respectively inserted into the upstream and downstream sections 911, 912 of the intestine 91, the clamping section 323 of the first unit 3 and the insertion member 41 of the second unit 4 cooperatively clamp the portion 919 of the intestine 91 therebetween for positioning the first and second units 3, 4 in the abdomen 9.

Specifically, the first and second units 3, 4 of the present embodiment are made of elastic materials, such as biocompatible medical grade silicones, to minimize discomfort for the patient. Thus, by exerting an external force onto the artificial stoma device, the clamping section 323 of the first unit 3 and the insertion member 41 of the second unit 4 would be further apart. Conversely, when the external force disappears, the clamping section 323 and the insertion member 41 would restore to be closer and to clamp the portion 919 of the intestine 91 therebetween.

Once the installation of the present embodiment is completed, the upstream section 911 of the intestinal 91, the inlet and outlet openings 310, 320 of the first unit 3, and the opening 920 of the skin tissue 92 are all in spatial communication such that an artificial stoma is formed. From here on, excrement transported through the upstream section 911 of the intestinal 91 is guided into the first unit 3 of the artificial stoma device via the inlet opening 310 of the inlet portion 31, and then discharged from the first unit 3 via the outlet opening 320 of the outlet portion 32. Also, in virtue of the second unit 4, no excrement is allowed to pass through the downstream section 912 of the intestinal 91 so that the downstream section 912 is allowed to rest until it restores to its normal functioning state.

The cover unit 5 is detachably coupled to the outlet portion 32 of the hollow member 30 of the first unit 3 and is adapted for blocking the opening 920 of the skin tissue 92. It should be noted that the cover unit 5 may be a piece of gauze, or any object that is sterilized and that has a shielding effect such that the cover unit 5 is able to protect the intestine 91 from direct exposure to the external environment, minimizing a risk of infection.

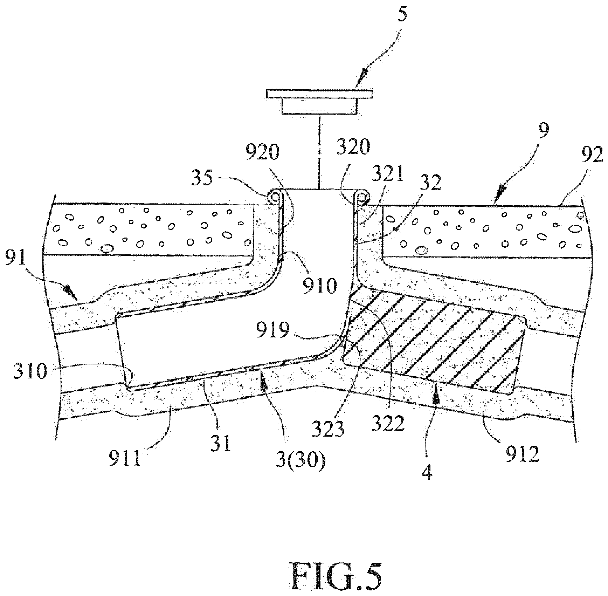

Referring to FIG. 5, a second embodiment of the present disclosure is similar to the first embodiment. The main difference between the two embodiments lies in that the first unit 3 of the second embodiment further includes a positioning member 35.

The positioning member 35 has an end that is connected to the outlet portion 32 of the hollow member 30 and that is adjacent to the outlet opening 320 of the outlet portion 32, and an opposite end that is adapted to be disposed at a periphery of the opening 920 of the skin tissue 92 and that is secured to the skin tissue 92. The cover unit 5 is detachably coupled to the positioning member 35 of the first unit 3 and is adapted for blocking the opening 920 of the skin tissue 92.

In virtue of the positioning member 35, the artificial stoma device of the second embodiment is more securely installed in the abdomen 9, and the cover unit 5 coupled to the positioning member 35 is able to provide the same shielding effect as in the previous embodiment.

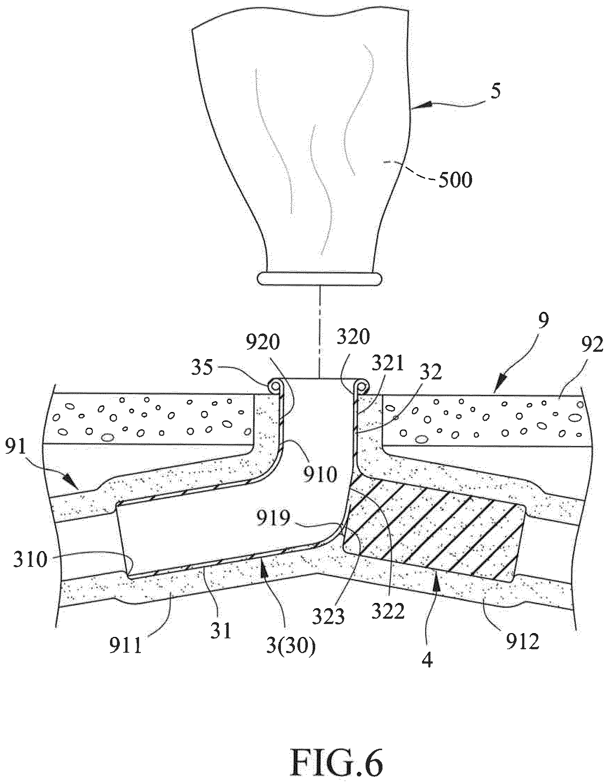

Referring to FIG. 6, a variation of the second embodiment is shown therein. In this variation, the cover unit 5 is a receiving bag. The receiving bag defines a receiving space 500 for receiving excrement discharged from the outlet opening 320 of the outlet portion 32, such that the cover unit 5 not only has the same shielding effect but serves as a temporary storage device. Once the excrement reaches a certain amount, the receiving bag may be detached and replaced to maintain hygiene and minimize the risk of infection.

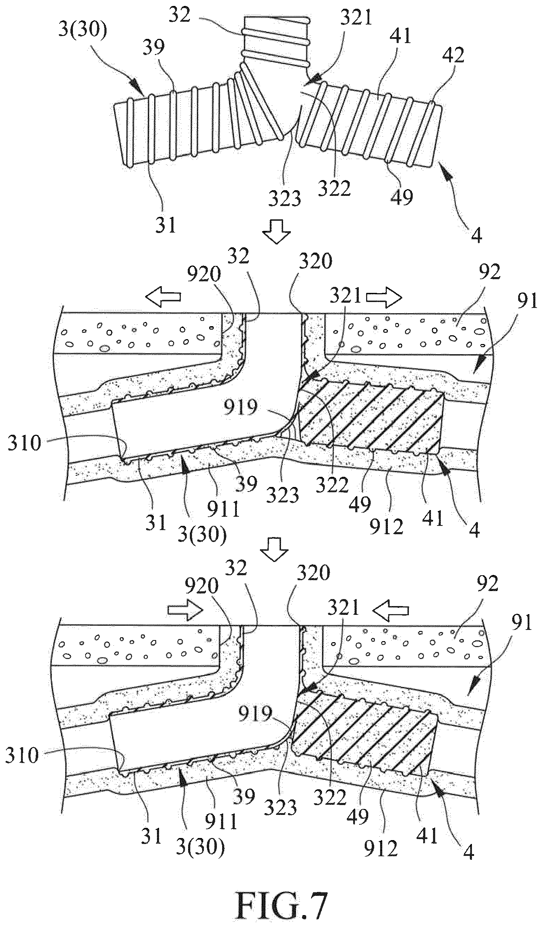

Referring to FIG. 7, a third embodiment of the present disclosure is similar to the first embodiment. The main difference between the two embodiments lies in that, in the third embodiment, the first unit 3 further includes a first protruding structure 39, and the second unit 4 further includes a second protruding structure 49.

The first protruding structure 39 is formed on an external surface of the hollow member 30 and is adapted to be in frictional contact with an inner surface of the intestine 91. The second protruding structure 49 is formed on an external surface of the insertion member 41 and is adapted to be in frictional contact with the inner surface of the intestine 91. In the present embodiment, the first and second protruding structures 39, 49 are helical structures.

In virtue of the first and second protruding structures 39, 49, the positioning of the first and second units 3, 4 is strengthened. Moreover, such configuration provides a massaging effect that helps stimulate muscles of the intestine 91 and facilitate the recovery of the intestine 91.

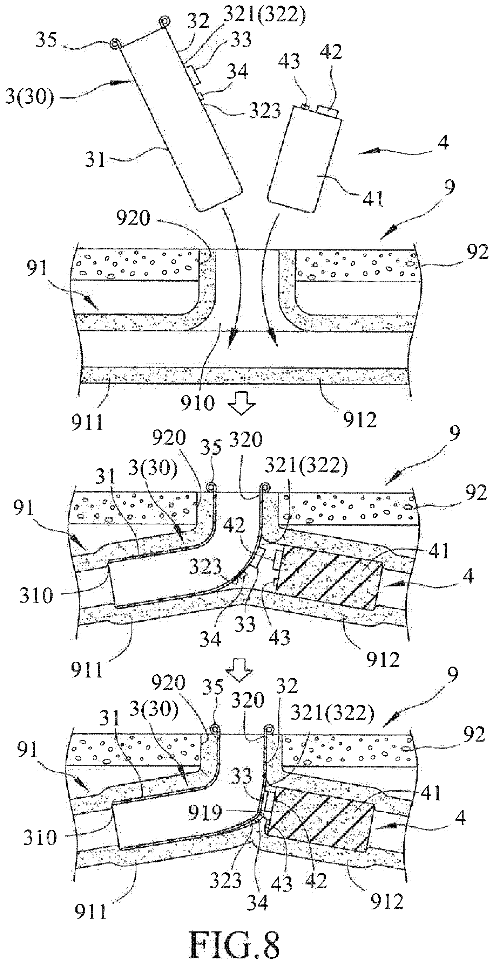

Referring to FIG. 8, a fourth embodiment of the present disclosure is similar to the first embodiment. The main difference between the two embodiments is described as follows.

In the present embodiment, the first unit 3 further includes a first connecting member 33 and a first fixing member 34. The first connecting member 33 is connected fixedly to the linking section 322 of the connecting segment 321 of the outlet portion 32. The first fixing member 34 is connected fixedly to clamping section 323 of the connecting segment 321 of the outlet portion 32, and is spaced apart from the first connecting member 33.

The second unit 4 further includes a second connecting member 42 and a second fixing member 43. The second connecting member 42 is connected fixedly to the insertion member 41 and is detachably connected to the first connecting member 33 of the first unit 3. The second fixing member 43 is connected fixedly to the insertion member 41, and is spaced apart from the second connecting member 42. The first and second fixing members 34, 43 are adapted to cooperatively clip the portion 919 of the intestine 91 therebetween.

It should be noted that prior to the installation of the present embodiment, the first and second units 3, 4 are separated. The first and second connecting members 33, 42 may be a mechanical fastener such as a buckle or a clasp, and the first and second fixing members 34, 43 may be two magnets.

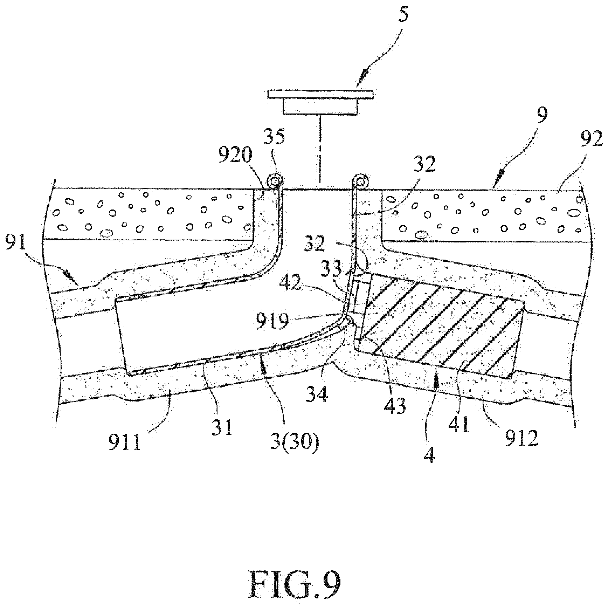

Referring to FIG. 9 in company with FIG. 8, the installation process of the present embodiment is similar to that of the first embodiment. After the first and second units 3, 4 are respectively inserted into the upstream and downstream sections 911, 912 of the intestine 91, the first connecting member 33 is connected to the second connecting member 42, and the first and second fixing members 34, 43 cooperatively clip the portion 919 of the intestine 91 therebetween for positioning the first and second units 3, 4 in the abdomen 9. The cover unit 5 of the present embodiment is a sealing cap which provides the same shielding effect as in the first embodiment.

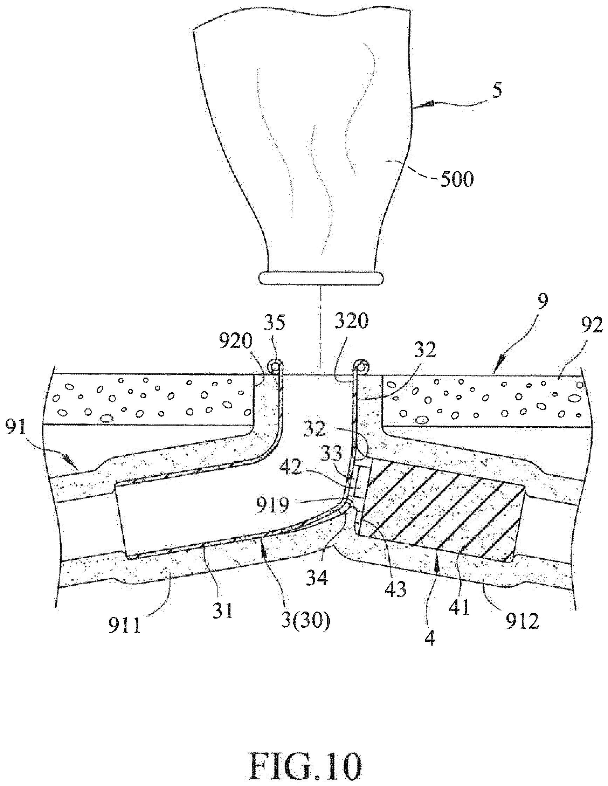

Referring to FIG. 10, a variation of the fourth embodiment is shown therein. Similar to the variation of the second embodiment, the cover unit 5 of the present variation is a receiving bag that defines a receiving space 500 for receiving excrement discharged from the outlet opening 320 of the outlet portion 32, such that the cover unit 5 provides the same shielding effect, as well as serving as a temporary storage device.

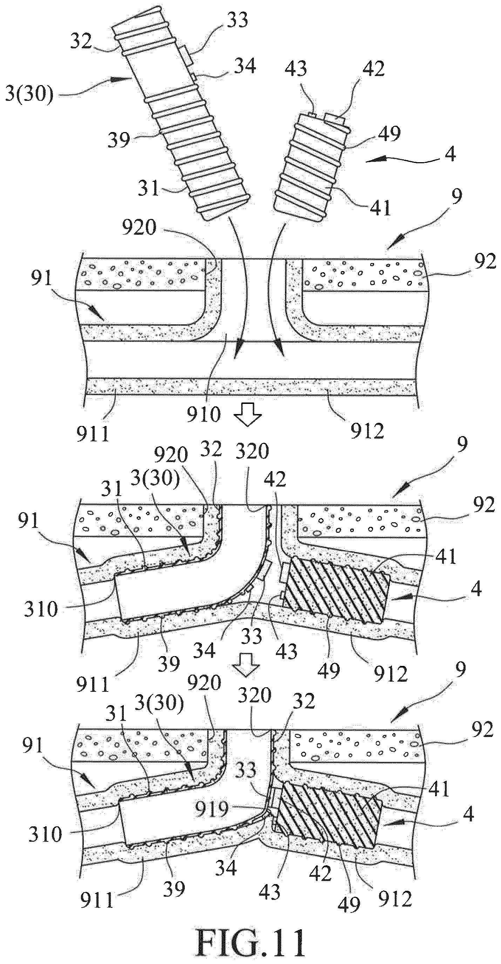

Referring to FIG. 11, a fifth embodiment of the present disclosure is similar to the fourth embodiment. The main difference between the two embodiments lies in that, in the fifth embodiment, the first unit 3 further includes a first protruding structure 39, and the second unit 4 further includes a second protruding structure 49.

Similar to the third embodiment, the first protruding structure 39 is formed on an external surface of the hollow member 30 and is adapted to be in frictional contact with an inner surface of the intestine 91. The second protruding structure 49 is formed on an external surface of the insertion member 41 and is adapted to be in frictional contact with the inner surface of the intestine 91. In the present embodiment, the first and second protruding structures 39, 49 are helical structures and are able to provide the massaging effect as mentioned above.

In sum, the artificial stoma device according to the present disclosure provides a more flexible design compared with the prior art. By virtue of the configurations of the first and second units 3, 4, the excrement transported through the upstream section 911 of the intestinal 91 is able to pass out of the intestine 91 without accidentally entering the downstream section 912 of the intestine 91. Thus, in cases where the downstream section 912 of the intestine 91 is not severed, the downstream section 912 is allowed to rest until it restores to its normal functioning state.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. An artificial stoma device adapted to be installed in an abdomen (9), the abdomen (9) containing an intestine (91) which is formed with a radial side opening (910), and having a skin tissue (92) which is formed with an opening (920) being registered with the radial side opening (910), the intestine (91) having an upstream section (911) that is disposed upstream of the radial side opening (910) and a downstream section (912) that is disposed downstream of the radial side opening (910), said artificial stoma device being **characterized by**:
a first unit (3) adapted to be inserted into the upstream section (911) of the intestine (91) via the opening (920) of the skin tissue (92) and the radial side opening (910) of the intestine (91), said first unit (3) including a hollow member (30) that has
an inlet portion (31) adapted to be disposed in the upstream section (911) of the intestine (91), and having an inlet opening (310) at a distal end thereof that is adapted for facing the upstream section (911) of the intestine (91), and
an outlet portion (32) adapted to be disposed at the opening (920) of the skin tissue (92) and the radial side opening (910) of the intestine (91), being connected to the inlet portion (31), and having an outlet opening (320) that is opposite to and in spatial communication with said inlet opening (310) and that is adapted to be exposed from the skin tissue (92) via the opening (920); and
a second unit (4) being connected to said first unit (3), and including an insertion member (41) that is adapted to be inserted into the downstream section (912) of the intestine (91) via the opening (920) of the skin tissue (92) and the radial side opening (910) of the intestine (91).

2. The artificial stoma device as claimed in claim 1, **characterized in that** said outlet portion (32) of said hollow member (30) of said first unit (3) has a connecting segment (321) that is adapted to be adjacent to the downstream section (912) of the intestine (91) and that is connected to said insertion member (41) of said second unit (4), said connecting segment (321) and said insertion member (41) being molded as one piece.

3. The artificial stoma device as claimed in claim 2, **characterized in that** said connecting segment (321) has:
a linking section (322) that is connected to said second unit (4); and
a clamping section (323) that cooperates with said second unit (4) for clamping a portion (919) of the intestine (91) therebetween.

4. The artificial stoma device as claimed in claim 1, **characterized in that**:
said first unit (3) further includes a first connecting member (33) that is connected fixedly to said hollow member (30); and
said second unit (4) further includes a second connecting member (42) that is connected fixedly to said insertion member (41) and that is detachably connected to said first connecting member (33) of said first unit (3).

5. The artificial stoma device as claimed in claim 4, **characterized in that**:
said first unit (3) further includes a first fixing member (34) that is connected fixedly to said hollow member (30), and that is spaced apart from said first connecting member (33);
said second unit (4) further including a second fixing member (43) that is connected fixedly to said insertion member (41), and that is spaced apart from said second connecting member (42); and
said first and second fixing members (34, 43) are adapted to cooperatively clip a portion (919) of the intestine (91) therebetween.

6. The artificial stoma device as claimed in any one of claims 1 to 5, further **characterized by** a cover unit (5) detachably coupled to said outlet portion (32) of said hollow member (30) of said first unit (3) and adapted for blocking the opening (920) of the skin tissue (92).

7. The artificial stoma device as claimed in any one of claims 1 to 5, **characterized in that** said first unit (3) further includes a positioning member (35) that has an end connected to said outlet portion (32) of said hollow member (30) and being adjacent to said outlet opening (320), and an opposite end adapted to be disposed at a periphery of the opening (920) of the skin tissue (92) and secured to the skin tissue (92).

8. The artificial stoma device as claimed in claim 7, **characterized by** a cover unit (5) detachably coupled to said positioning member (35) of said first unit (3) and adapted for blocking the opening (920) of the skin tissue (92).

9. The artificial stoma device as claimed in any one of claims 1 to 8, **characterized in that**:
said first unit (3) further includes a first protruding structure (39) formed on an external surface of said hollow member (30) and adapted to be in frictional contact with an inner surface of the intestine (91); and
said second unit (4) further includes a second protruding structure (49) formed on an external surface of said insertion member (41) and adapted to be in frictional contact with the inner surface of the intestine (91).

10. The artificial stoma device as claimed in claim 9, **characterized in that** said first and second protruding structures (39, 49) are helical structures.

## Amended claims

### Amended claims in accordance with Rule 137(2) EPC.

1. An artificial stoma device adapted to be installed in an abdomen (9), the abdomen (9) containing an intestine (91) which is formed with a radial side opening (910), and having a skin tissue (92) which is formed with an opening (920) being registered with the radial side opening (910), the intestine (91) having an upstream section (911) that is disposed upstream of the radial side opening (910) and a downstream section (912) that is disposed downstream of the radial side opening (910), said artificial stoma device including
a first unit (3) adapted to be inserted into the upstream section (911) of the intestine (91) via the opening (920) of the skin tissue (92) and the radial side opening (910) of the intestine (91), said first unit (3) including a hollow member (30) that has
an inlet portion (31) adapted to be disposed in the upstream section (911) of the intestine (91), and having an inlet opening (310) at a distal end thereof that is adapted for facing the upstream section (911) of the intestine (91), and
an outlet portion (32) adapted to be disposed at the opening (920) of the skin tissue (92) and the radial side opening (910) of the intestine (91), being connected to the inlet portion (31), and having an outlet opening (320) that is opposite to and in spatial communication with said inlet opening (310) and that is adapted to be exposed from the skin tissue (92) via the opening (920), and
a second unit (4) being connected to said first unit (3), and including an insertion member (41) that is adapted to be inserted into the downstream section (912) of the intestine (91) via the opening (920) of the skin tissue (92) and the radial side opening (910) of the intestine (91),
said outlet portion (32) of said hollow member (30) of said first unit (3) having a connecting segment (321) that is adapted to be adjacent to the downstream section (912) of the intestine (91) and that is connected to said insertion member (41) of said second unit (4), said connecting segment (321) and said insertion member (41) being molded as one piece,
said artificial stoma device being **characterized in that** :
said connecting segment (321) has
a linking section (322) that is connected to said second unit (4), and
a clamping section (323) that cooperates with said second unit (4) for clamping a portion (919) of the intestine (91) therebetween.

2. The artificial stoma device as claimed in claim 1, further comprising a cover unit (5) detachably coupled to said outlet portion (32) of said hollow member (30) of said first unit (3) and adapted for blocking the opening (920) of the skin tissue (92).

3. The artificial stoma device as claimed in any one of claims 1 and 2, wherein said first unit (3) further includes a positioning member (35) that has an end connected to said outlet portion (32) of said hollow member (30) and being adjacent to said outlet opening (320), and an opposite end adapted to be disposed at a periphery of the opening (920) of the skin tissue (92) and secured to the skin tissue (92).

4. The artificial stoma device as claimed in claim 3, comprising a cover unit (5) detachably coupled to said positioning member (35) of said first unit (3) and adapted for blocking the opening (920) of the skin tissue (92).

5. The artificial stoma device as claimed in any one of claims 1 to 4, wherein:
said first unit (3) further includes a first protruding structure (39) formed on an external surface of said hollow member (30) and adapted to be in frictional contact with an inner surface of the intestine (91); and
said second unit (4) further includes a second protruding structure (49) formed on an external surface of said insertion member (41) and adapted to be in frictional contact with the inner surface of the intestine (91).

6. The artificial stoma device as claimed in claim 5, wherein said first and second protruding structures (39, 49) are helical structures.
